(19) Europäisches Patentamt
European Patent Office
Office européen des brevets

(11)  EP 2 688 548 B1

(12)  **EUROPEAN PATENT SPECIFICATION**

(45) Date of publication and mention
of the grant of the patent:
**01.02.2017   Bulletin 2017/05**

(51) Int Cl.:
*A61Q 19/08* (2006.01)          *A61K 8/73* (2006.01)
*A61Q 19/00* (2006.01)

(21) Application number: **12711124.3**

(22) Date of filing: **16.03.2012**

(86) International application number:
**PCT/EP2012/054708**

(87) International publication number:
**WO 2012/130642 (04.10.2012 Gazette 2012/40)**

(54) **SKIN CARE COMPOSITION**

HAUTPFLEGEZUSAMMENSETZUNG

COMPOSITION DE SOINS DE BEAUTÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **25.03.2011   IT VA20110009**

(43) Date of publication of application:
**29.01.2014   Bulletin 2014/05**

(73) Proprietor: **Lamberti SpA
21041 Albizzate (VA) (IT)**

(72) Inventors:
• **FUMAGALLI, Chiara
I-21010 Besnate (VA) (IT)**
• **BALDARO, Eva
I-20127 Milano (IT)**
• **LANGELLA, Valentina
I-20155 Milano (MI) (IT)**
• **TENCONI, Mauro
I-21045 Gazzada (VA) (IT)**
• **PELIZZARI, Raffaella
I-20013 Magenta (MI) (IT)**
• **FLORIDI, Giovanni
I-28100 Novara (IT)**

• **LI BASSI, Giuseppe
I-21026 Gavirate (VA) (IT)**

(74) Representative: **Giaroni, Paola
LAMBERTI S.p.A.
Ufficio Brevetti
Via Piave, 18
21041 Albizzate (VA) (IT)**

(56) References cited:
**EP-A1- 1 559 417      FR-A1- 2 881 349
US-B2- 6 884 884**

• **JOUANDEAUD M ET AL: "THE INFLUENCE OF
OLIGOSACCHARIDES ON SKIN AGING: AN
ALTERNATIVE TO RETINOIDS", COSMETICS &
TOILETRIES, WHEATON, IL, US, vol. 119, no. 6, 1
June 2004 (2004-06-01), pages 67-74,76,
XP008034377, ISSN: 0361-4387**
• **YU CHENG ET AL: "Characterization and
Intermolecular Interactions of Hydroxypropyl
Guar Solutions", BIOMACROMOLECULES, vol. 3,
no. 3, 1 May 2002 (2002-05-01), pages 456-461,
XP055011579, ISSN: 1525-7797, DOI:
10.1021/bm0156227**

**Description**

*Technical Field*

The present invention relates to a cosmetic composition for enhancing the overall quality of the skin and for treating aging skin, comprising from 0.05 to 5.0 % by weight of a depolymerized hydroxypropyl guar having MS comprised between 1.5 and 3.5, in a cosmetic or pharmaceutical acceptable carrier.

*Prior art*

In all living bodies, including human beings, organs, such as the skin, gradually deteriorate as they grow old. As the skin ages, protection of the skin against stimulation, such as oxidation stress, weakens to cause the conditions inside the skin to be bad, thus promoting the aging. Several factors contribute to the aging of skin, leading to accelerated deterioration evidenced by wrinkles and sagging of the skin, for example the excessive exposure to ultraviolet radiation and sunlight causes skin to look prematurely aged. As a result of skin aging, deep changes happen at both dermis and epidermis levels, in particular, the skin loses its elasticity: the skin does not snap back when pinched and wrinkles, stretch marks and sagging are more obvious as result of free-radical on the body.

The connective tissue of the skin is composed mostly of collagen and elastin. Collagen gives the dermis its mechanical and structural integrity, while elastin provide the elasticity of the skin. The first and most notable sign of aging is a decrease in the water retention capability of the skin and the resultant decrease in dermis elasticity. Moreover during menopause the elastin fibers atrophy as dermis thins and their production is altered; thus the dermis loses its viscoelastic properties.

A major concern in the improvement of skin care has been treating the signs of aging, especially on the face and on the part of the body more subjected to stress. As such, there are many products on the market directed towards fighting various skin conditions associated with loss of skin elasticity and firmness, folds in the eyelids, skin sagging, aging signs such as fine lines and wrinkles and so on.

The addition of collagen or elastin, both in native and hydrolyzed form, does not provide an effect on skin elasticity, but collagen and elastin show filming, conditioning and hydrating properties, thus finding application in anti-age products. Moreover, they are extracted from animals, typically young bovines, and in personal care there is the trend to decrease the use of products of animal origin.

Hyaluronic acid, a successful polysaccharidic anti-age ingredient, acts on the hydration of the skin and not on its elasticity.

Products like vegetal proteins, hydrolyzate peptides extracted from plants or marine algae, phytosterols, polyphenols and flavonoids from plants or plant stem cells, plant extracts of several origins are well accepted by the consumers and also provide some effect on the skin elasticity. However, since skin care needs vary from person to person, no single products suits every individual. Thus, there is always a need for improved products, especially compositions, which are effective in the treatment of skin conditions associated with aging, coming from non-animal sources and with a good compatibility for sensitive skin types.

The inventors of the present invention have now discovered, surprisingly and unexpectedly, that a nonionic depolymerized hydroxypropyl guar with high molar substitution increases the elasticity of skin and can be used as an anti-aging ingredient for personal care application.

Hydroxypropyl guar (HPG) is prepared from guar gum, a polygalactomannan, with a ratio mannose/galactose of about 2/1, by reaction with propylene oxide under basic conditions. The introduction of hydroxypropyl groups renders guar more organophilic, especially at high molar substitution, increasing its solubility in organic solvents such as alcohols and glycols.

Several studies describe methods for depolymerizing polysaccharides and in particular polygalactomannans, such as guar and guar derivatives. We can cite, for example, US 3,728,331, US 4,874,854, US 5,708,162, US 4,753,659, US 6,884,884, EP 030443, EP 1417240, JP 7100017, WO 93/15116, WO 99/04027, or Craig, D., et al. (Proceedings of the Thirty-Ninth Annual Southwestern Petroleum Short Course, Southwestern Petroleum Short Course Association, Inc., Apr. 22-23, 1992, Texas Tech University, Lubbock, Texas), Vijayendran and Bone (Carbohydrate Polymers 1994, 4: 299-313), Frollini, E. et al. (Carbohydrate Polymers 1995, 27: 129-135), Ouchi, T., et al. (J.M.S.-Pure Appl. Chem. 1997, A34(6): 975-989), Tayal, A., et al. (Macromolecules 2000, 33:9488-9484). The resulting depolymerized compositions have been utilized in different fields such as oil drilling, building industry, food industry, paper industry, agrochemicals etc. Hydroxypropyl guar derivatives are also widely used in the cosmetic field as nonionic polymeric thickeners and film formers. It is often specified in personal care formulations that they take advantage from their characteristics, such as high level of lubricity as well as excellent salt and alcohol tolerance in aqueous solutions.

Nonetheless, as far as the Applicant knows, none has described the effects of a depolymerized HPG with high molar substitution on the human skin. The discovery of these effects has allowed to propose a particularly original solution for improving the elasticity of skin. This solution consists in applying, to the parts of the skin to be treated, an effective amount of a composition comprising depolymerized HPG with high molar substitution.

In the present text, with the expression "molar substitution" (MS), we mean the number of hydroxypropyl groups on each monosaccharidic unit of guar which can be measured, for example, by $^1$H-NMR.

With "depolymerized hydroxypropyl guar" we mean a hydroxypropyl guar whose average molecular weight has been sensibly reduced using a degrading treatment.

*Description of the Invention*

It is therefore an object of the present invention a skin care composition comprising from 0.05 to 5.0% by weight (wt) of a depolymerized hydroxypropyl guar having molar substitution comprised between 1.5 and 3.5 and RVT Brookfield® viscosity at 10 % by weight in water, 20 °C, 20 rpm from 50 to 5000 mPa*s together with an acceptable cosmetic or pharmaceutical carrier or diluent.

It is another object of the invention the use of a depolymerized hydroxypropyl guar having molar substitution comprised between 1.5 and 3.5 and RVT Brookfield® viscosity at 10 % by weight in water, 20 °C, 20 rpm from 50 to 5000 mPa*s for increasing the elasticity of the skin.

*Detailed Description of the Invention*

According to a preferred embodiment of the present invention, the depolymerized hydroxypropyl guar has molar substitution comprised between 2.0 and 3.2.

In a particularly preferred embodiment the HPG of the invention exhibits a RVT Brookfield® viscosity comprised between 100 and 2000 mPa*s at 20°C, 20 rpm and 10 % by weight in water.

The depolymerized HPG with high MS of the invention can be prepared by using any of the methods known in the art, such as those described in the literature cited before.

In a preferred embodiment, the depolymerized HPG with high MS of the invention is obtained by reducing the molecular weight of a hydroxypropyl guar with high MS.

Hydroxypropyl derivatives of guar are obtained by chemical reaction of the hydroxyl groups of the polygalactomannan chain with propylene oxide, in the presence of an alkaline catalyst (such as sodium hydroxide), according to procedures well known to the man skilled in the art. Specific details can be found for example in "Industrial Gums: Polysaccharides and their Derivatives", 3rd Ed., Whistler, Roy, L, and BeMiller, James N., Academic Press (1993).

"Guar gum" or simply "Guar" consists of a main linear chain of mannose units bearing branches of galactose units in a molar ratio of about 2:1 and is made by the thermo-mechanical treatment of the seeds of "Cyamopsis Tetragonolobus", a leguminosae cultivated in the semi-dry regions of tropical countries, particularly in India and in Pakistan. It is usually found in the form of "splits", that are the endosperms of the seed deprived of the husk and from the inner part, the germ, or in the form of powder (flour) of different particle-size, which is obtained from the splits by milling.

The derivatization process with propylene oxide is applicable indifferently to guar in the form of flour or in the form of "splits".

At the end of the derivatization process HPG has usually an average molecular weight comprised between 500,000 and 2,000,000. Da and RVT Brookfield® viscosity between 1,000 and 30,000 mPa*s at a concentration of 2% by weight in water, 20 °C and 20 rpm.

Before being depolymerized, the HPG with high MS used for the realization of the invention can be purified with methods well known in the art from the by-products generated during the chemical reaction (glycols, polyglycols, inorganic/organic salts), for example washing with water or an organic solvent, or a mixture of both.

The HPG with high MS can be depolymerized by oxidation, for example with alkali or hydrogen peroxide, or by other depolymerization reactions, such as enzymatic or thermal depolymerisation, or acid hydrolysis. The depolymerized HPG used in this invention is preferably prepared by acid hydrolysis of hydroxypropyl guar in water solution.

At the end of the process, the depolymerized HPG with high MS can be left in the reaction medium or can be recovered using methods known in the art, such as by filtration, addition of solvents, freeze drying and the like.

In another preferred embodiment, the depolymerized HPG with high MS of the invention is prepared by reducing the molecular weight of guar first, then by derivatizing with propylene oxide. Both reactions can be performed using the already mentioned methods.

The average molecular weight of the depolymerized HPG with high MS of the invention is comprised between 2,000 to 200,000 Da.

Suitable cosmetic or pharmaceutical acceptable carrier and diluents are well known in the art and can be of a variety of forms. They can be solvents or dispersion mediums containing, for example, water, ethanol, polyols (for example glycerol, propylene glycol, liquid polyethylene glycol and the like), oils and suitable mixtures thereof. The typical carrier/diluent can be in the form of an aqueous or hydro-alcoholic system, an emulsion or a gel; emulsions also include microemulsion systems.

When the composition is provided in emulsion form, this emulsion requires the presence of an emulsifier and oils (water insoluble) which are well known in the art.

The skin care composition in emulsion form of the invention can comprise from 0.1 to 15.0 % by weight of an emulsifier and from 1.0 to 60 % by weight of an oil.

Oils include hydrocarbon oils and waxes, silicone oils, fatty acid derivatives, cholesterol, cholesterol derivatives, diglycerides, triglycerides, vegetable oils, vegetable oil derivatives, alkyl or alkenyl esters, lanolin and its derivatives, wax esters, beeswax derivatives and sterols and combinations thereof.

Examples of hydrocarbon oils and waxes suitable for use herein include petrolatum, mineral oil, micro-crystalline waxes, polyalkenes, paraffins, cerasin, polyethylene, perhydrosqualene, poly alpha-olefins, hydrogenated polyisobutenes and combinations thereof.

Examples of silicone oils suitable for use herein include dimethicone copolyol, dimethylpolysiloxane, diethyl-polysiloxane, mixed $C_1$-$C_{30}$ alkyl polysiloxanes, phenyl dimethicone, dimethiconol and combinations thereof.

Examples of diglycerides and triglycerides suitable for use herein include castor oil, soy bean oil, derivatized soybean oils such as maleated soy bean oil, safflower oil, cottonseed oil and derivatized cottonseed oil, corn oil, walnut oil, peanut oil, olive oil, cod liver oil, almond oil, palm oil and sesame oil, sunflower seed oil, coconut oil and derivatized coconut oil, cocoa butter, and combinations thereof.

Examples of alkyl esters suitable for use herein include cetyl ricinoleate, stearyl ricinoleate, hexyl laurate, isohexyl laurate, myristyl myristate, decyl oleate, isodecyl oleate, hexadecyl stearate, decyl stearate, isopropyl isostearate, dihexyldecyl adipate, diisopropyl sebacate, lauryl lactate, myristyl lactate, cetyl lactate, and combinations thereof.

Examples of alkenyl esters suitable for use herein include oleyl myristate, oleyl stearate, oleyl oleate, and combinations thereof.

Examples of lanolin and lanolin derivatives suitable for use herein include lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol ricinoleate, hydroxylated lanolin, hydrogenated lanolin and combinations thereof.

Other suitable oils include milk triglycerides (e.g., hydroxylated milk glyceride) and polyol fatty acid polyesters.

Still other suitable oils include wax esters, vegetable waxes and sterols, non-limiting examples including beeswax and beeswax derivatives, stearyl stearate; carnauba and candelilla waxes; cholesterol, ceramides; and combinations thereof.

Suitable emulsifiers are well known in the art and include nonionic, anionic, amphoteric, zwitterionic, cationic emulsifier and mixtures thereof. Examples of emulsifiers include also natural or synthetic polymeric emulsifiers. Anionic emulsifier include alkyl and alkyl ether sulfates, alkyl sulfonates, alkyl and alkyl ether phosphates, alkyl or alkyl ether sulfosuccinates, alkyl and alkyl ether carboxylates and anionic derivatives of alkyl polyglycosides, such as the citric, tartaric or sulfosuccinic ester of alkyl polyglucosides. Nonionic emulsifiers can be broadly defined as compounds containing a hydrophobic moiety and a nonionic hydrophilic moiety. Examples of the hydrophobic moiety can be alkyl, alkyl aromatic, and aryl aromatic. Examples of hydrophilic moieties are polyoxyalkylenes, amine oxides, and alkanol amides. Examples of non-ionic emulsifiers are alkoxylated fatty alcohols or fatty acids, alkoxylated di- and tri-stiryl phenols, polyhydroxy fatty acid amides, sugar esters and polyesters, alkoxylated sugar esters, sorbitan and alkoxylated sorbitan fatty acid esters. Other examples of nonionic emulsifiers include alkyl polyglycosides, such as coco polyglucosides.

Cationic emulsifiers useful in the composition of the present invention contain amino or quaternary ammonium hydrophilic moieties which are positively charged when dissolved in water. Examples of ammonium compounds are long-chain alkyl trimethyl ammonium chloride, long-chain alkyl benzyl dimethyl ammonium chloride, alkylamine hydrochlorides, alkylamine acetates and di(long-chain alkyl) dimethyl ammonium bromide.. The amphoteric emulsifiers which can be used in the composition of the present invention are those which can be broadly described as derivatives of aliphatic quaternary ammonium compounds, wherein one of the aliphatic substituents contains an anionic water-solubilizing group, e.g., carboxylate, sulfonate, sulfate. Examples of amphoteric surfactants include cocoamphocarboxypropionate, cocoamphoacetate, cocoamphodiacetate, sodium lauroamphoacetate.

Examples of zwitterionic surfactants include alkyl betaines and amido betaines, alkyl sultaines, alkyl glycinates and alkyl carboxyglycinates.

Polymeric emulsifiers that are suitable for use herein include, but are not limited to, carboxylic acid polymers which are crosslinked compounds containing one or more monomers derived from acrylic acid, substituted acrylic acids, and salts and derivatives of these acrylic acids and substituted acrylic acids. These carboxylic acid polymers also act as thickening agents. They can be crosslinked homopolymers of a acrylic acid or of a derivative thereof, such as acrylamidopropylsulfonic acid. They can be also crosslinked copolymers having (i) a first monomer selected from the group consisting of (meth)acrylic acid, derivatives thereof, short chain (i.e. $C_1$-$C_4$) acrylate ester monomers, and mixtures thereof; and (ii) a second monomer which is a long chain (i.e. $C_8$-$C_{40}$) substituted polyethylene glycol acrylate ester monomer.

Examples of commercially available carboxylic acid polymers useful herein are Carbopol 1342, Pemulen TR-1, Pemulen TR-2 (from Lubrizol Corp.); Sepigel 305, Simulgel EG, Simulgel NS, Simulgel 600 (from Seppic S.A.); Viscolam AT100P and Viscolam AT64/P (from Lamberti S.p.A.).

Other materials that may be suitable as polymeric emulsifiers include ethylene oxide/propylene oxide block copolymers, for example those commercialized under the trade name Pluronic (BASF).

Other suitable polymeric emulsifiers include natural polymer derivatives such as polysaccharides that may be derivatized with hydrophobic moieties. Further examples of suitable emulsifiers that can be used in the composition of the present invention are disclosed in "McCutcheon's Detergents and Emulsifiers", North American Edition (2003), Allured Publishing Corporation. The composition of the invention can comprise additional cosmetically-functional agents. The term "cosmetically-functional agent", as used herein, means any material, compound or composition which can be applied to skin for cosmetic scope. Non-limiting examples of these agents that may be included in the skin care composition according to the present invention are the following:

- anti-wrinkle agents, such as retinol, hydroxy acids (e.g., alpha-hydroxy acids such as lactic acid and glycolic acid or beta-hydroxy acids such as salicylic acid and salicylic acid derivatives such as the octanoyl derivative) and tocopherols;
- anti-oxidants/radical scavengers, such as ascorbic acid and its salts, ascorbyl esters of fatty acids, ascorbic acid derivatives, tocopherol, tocopherol acetate, other esters of tocopherol, gallic acid and its alkyl esters, uric acid and its salts and alkyl esters, sorbic acid and its salts, and the like;
- skin coolants, such as menthol, menthyl acetate, and other derivatives of menthol, which give rise to a tactile response in the form of a cooling sensation on the skin;
- emollients, such as isopropylmyristate, $C_{12}$-$C_{15}$ alkyl benzoate, silicone materials, mineral oils and vegetable oils which give rise to a tactile response in the form of an increase in skin lubricity;
- skin bleaching and lightening agents, such as hydroquinone, kojic acid, arbutin, ascorbic acid and derivatives thereof, extracts (e.g., mulberry extract, placental extract) as well as titanium dioxide and zinc oxide.
- sunscreen agents, among which inorganic sunscreen agents (for example metallic oxides such as titanium dioxide, zinc oxide, zirconium oxide, iron oxide and mixtures thereof) and organic sunscreen agents (for example p-aminobenzoic acid, its salts and its derivatives, anthranilates, benzalacetophenone, benzophenones, cinnamic acid derivatives, coumarin derivatives, dihydroxycinnamic acid derivatives, trihydroxy-cinnamic acid derivatives, hydrocarbons, dibenzalacetone, naphtholsulfonates, di-hydroxynaphthoic acid and its salts, salicylates, quinine salts, quinoline derivatives, hydroxy- or methoxy-substituted benzophenones, uric and violuric acids, tannic acid and its derivatives, hydroquinone, octocrylene, and mixture thereof;
- tanning agents, such as dihydroxyacetone, which is also known as DHA or 1,3-dihydroxy-2-propanone;
- skin soothing and/or healing agents, such as panthenoic acid derivatives, for example panthenol, dexpanthenol and ethyl panthenol, aloe vera, retinoids, vitamins and derivatives thereof;
- moisturizing agents, that keep the skin moist by either adding moisture or preventing moisture from evaporating from the skin;
- topical anesthetics, such as benzocaine, lidocaine, chlorprocaine, dibucaine, etidocaine, tetracaine, procaine, ketamine, pramoxine, phenol, pharmaceutically acceptable salts thereof, and combinations thereof;
- perfumes, which give rise to an olfactory response, in the form of a fragrance or deodorant perfumes, which also reduce body malodor;
- deodorants other than perfumes, whose function is to reduce the level of or to eliminate micro flora at the skin surface, especially those responsible for the development of body malodor.
- beauty aids - such as foundation powders;
- shaving actives;
- anti-acne agents, such as resorcinol, sulfur, salicylic acid, benzoyl peroxide, erythromycin, zinc, and other similar materials;
- chelating agents - such as furildioxime, furilmonoxime, and derivatives thereof;
- flavonoids suitable for use on the skin as skin benefit agents, such as unsubstituted flavanones, mono-substituted flavanones, chalcones, flavones, coumarins and mixtures thereof;
- steroidal or nonsteroidal anti-inflammatory agents;
- antimicrobial agents, such as norfloxacin, tetracycline, ethambutol, erythromycin, phenoxyethanol, phenoxy propanol, methacycline, phenoxyisopropanol, chlorhexidine, chlortetracycline, oxytetracyiine, hexamidine isethionate, metronidazole, streptomycin, tetracycline hydrochloride, oxytetracycline, zinc pyrithione, and combinations thereof;
- visual skin enhancers, that mask the appearance of any number of skin imperfections such as age spots, fine lines, wrinkles and blemishes, for example titanium dioxide, zinc oxide and iron oxides and organic particulates that diffuse light when deposited on the skin.

The additional cosmetically-functional agents can be used in concentrations from 0.01 to 30 %by weight of the skin care composition.

The above lists of cosmetically-functional agents are only examples and are not a complete lists of ingredients that can be used. Other agents that can be used in these types of products are well known in the cosmetic industry. In addition to the above cosmetically-functional agents, the composition according to the present invention can optionally also include other additives, which are conventionally used in the cosmetic industries, such as colorants, preservatives (e.g. imidazolidinyl urea, diazolidinyl urea, phenoxyethanol, methylparaben, ethylparaben, propylparaben, etc.), antifoaming agents, nutritional supplements, activity enhancer, solubilizing agents, functional polymers, thickening agents, stabilizers, suspending agents (such as clays, silica and xanthan), silicone material, hydrocarbon polymers, medicaments, and mixtures thereof.

Examples of thickening agents that can be used in the composition of this invention include fatty alcohols; fatty acid esters; fatty acid amides; clays; silicas; anionic, cationic, hydrophobically-modified and amphoteric acrylic copolymers; nonionic, cationic, anionic and amphoteric cellulosic polymers (such as hydroxyethyl cellulose, hydroxypropyl cellulose, carboxymethyl cellulose and cationic hydroxypropyl cellulose); nonionic, anionic, hydrophobically-modified, amphoteric and cationic polysaccharides (such as xanthan, chitosan, carboxymethyl guar, hydroxypropyl guar, cationic hydroxypropyl guar).

Suitable stabilizers include (meth)acrylic copolymers; polycarbonates; polyethers, such as polyoxyethylene/polyoxypropylene block polymer; polyethylenes; polypropylenes; polyvinyl chloride; polystyrene; polyamides; cyclodextrins; mixtures thereof.

The pH of the composition is an important factor in the stability of the skin care composition of the invention. Preferably the pH range is between 3.5 and 9.0. A wide variety of acids, bases, and buffers can be utilized to adjust and/or maintain the pH of the compositions of the present invention. Examples of materials useful for adjusting and/or maintaining the pH include, without limitation, ammonia, sodium carbonate, sodium hydroxide, triethanolamine, hydrochloric acid, phosphoric acid, sodium hydrogen phosphate, sodium dihydrogen phosphate, citric acid, and the like.

Skin elasticity is critical for improving damage such as sagging, reduced skin firmness and lack of youthful appearance, and improving the appearance of fine lines and wrinkles. The use of the depolymerized HPG with high MS of the invention in compositions to be applied on the skin allows to increase the skin elasticity and , in particular, in combination with other well-known cosmetically-functional agents can help to treat, reverse, and/or prevent signs of skin aging.

The skin care compositions according to the invention can be formulated as skin lotions, skin creams, body butters, after-shower lotions, after-cleansing lotions, cleansing milk, after-shave products, deodorant products, antiperspirant products, sun care preparations and self-tanning creams. Preferred skin care composition are skin creams such as anti-age creams (anti-age day creams, anti-age night creams, eye contour creams, lip contour creams), anti-cellulite creams, anti-stretch mark creams (for prevention and reduction) and scar reduction creams; sun care preparations (sunscreens and after-sun) and hand care creams.

For a more detailed disclosing of the invention, reference can be made to the following examples which are intended as further illustration of the invention and are not to be construed in a limiting sense. All parts and percentages are by weight (wt) unless otherwise stated.

EXAMPLES

Example 1

A cream according to the invention was prepared, using the ingredients reported in Table 1 and according to the following manufacturing procedure:

Table 1

| INGREDIENTS | | % wt |
|---|---|---|
| **PHASE A** | | |
| 1 | Stearic Acid | 3 |
| 2 | Cetearyl Alcohol | 3 |
| 3 | Ceteareth-25 | 2 |
| 4 | Ceteareth-6 | 2 |
| **PHASE B** | | |
| 5 | Water | to 100 |

(continued)

| PHASE B | | |
|---|---|---|
| 6 | Depolymerised HPG*, 10% wt water solution | 20.0 |
| PHASE C | | |
| 7 | Diazolinidyl Urea, Methylparaben, Propylparaben | 0.5 |
| 8 | Parfum | 0.3 |
| 9 | Sodium Hydroxyde, 10% water solution | to pH 6.0-6.5 |
| * depolymerized HPG having MS = 2.6 and RVT Brookfield® viscosity of 300 mPa*s (10% wt in water at 20 rpm at 20 °C) | | |

Manufacturing procedure: in the main vessel the ingredients of Phase B are weighed and heated to 70-75°C; in a side vessel the ingredients of Phase A are weighed and heated to 70-75°C. Once the temperature is reached, the two mixtures are mixed and stirred until an emulsion is formed (about 5 min). The emulsion is then cooled to 30 °C and the ingredients 7 and 8 of Phase C are added. Finally, the pH is adjusted to a value of 6.0 - 6.5 with the 10% sodium hydroxide solution.

Example 2 (comparative)

A cream (placebo) was prepared without the depolymerized HPG using the same ingredients and manufacturing procedure of Example 1.

Example 3

Skin elasticity evaluation

The elasticity measurements are performed with a Cutometer® SEM 575, (Courage & Khazaka). In elastometric measurement, the skin surface is aspirated from the depression induced by the machine into the aperture of the elastometer's measuring probe. The depth of the skin penetration inside the probe is measured by an optic sensor. The total deformation of the skin obtained at the end of an aspiration cycle is defined as skin extensibility ($U_f$) During the releasing phase, the quantity of total deformation recovery can be observed ($U_a$= deformation recovery).
Cutaneous elasticity (or overall elasticity) is defined as the ratio:

$$U_a/U_f$$

The overall elasticity, whose values range between 0 and 1 (maximum elasticity), reflects the skin's potential capacity for retraction.
For this test, a suction cycle of 1 second at 350 mbar vacuum followed by a release cycle of one second was selected. Three measurement cycles (1 cycle: suction/release) are performed on the same point.
The three suction/release cycles may be represented as three successive curves, which provide the mean values of the deformation parameters relating to the elastic features of the skin. 12 caucasian woman aged from 40 to 60 (mean age 50.5 years) were included in this study.
The study was carried out in a bioclimatic room (24 +/- 2 °C; 50 +/- 10 % rh). The volunteers were asked not to apply any product on the face for at least 12 hours before performing the instrumental measurements.

The first day of the test the initial instrumental measurement of overall elasticity (To) was performed.

Immediately after, 2mg/cm$^2$ of each cream of Example 1 and 2 were applied on each half face of the volunteers.
The overall elasticity measurements were repeated 15 minutes after the products application ($T_{15}$).
The treatment was repeated by each woman twice a day for 2 weeks. At the end of the treatment period the volunteers came back to the laboratory to repeat the final measurement of overall elasticity ($T_f$).
Mean values and standard deviations for the overall elasticity values To, $T_{15}$ and $T_f$ are reported in Table 2.

Furthermore, the difference between $T_{15}$ or $T_f$ and $T_0$ were calculated and reported in Table 3 together with the percentage of variation.

The *t* test (Table 3) was used to determine the significance of differences detected before and after treatment. Differences at $p \leq 0.05$ were considered statistically significant.

Table 2

| OVERALL ELASTICITY | | $T_0$ | $T_{15}$ | $T_f$ |
|---|---|---|---|---|
| Example 2* | Mean | 0.504 | 0.528 | 0.463 |
| | Std. Dev. | 0.142 | 0.145 | 0.096 |
| Example 1 | Mean | 0.489 | 0.517 | 0.549 |
| | Std. Dev. | 0.113 | 0.112 | 0.115 |
| * Comparative | | | | |

Table 3

| SAMPLE | Elasticity $T_{15}$ - $T_0$ | | | Elasticity $T_f$ - $T_0$ | | |
|---|---|---|---|---|---|---|
| | $\Delta$ | % $\Delta$ | t-test | $\Delta$ | % $\Delta$ | t-test |
| Example 2* | 0.024 | 4.8 | p > 0.05 | -0.041 | -8.1 | p > 0.05 |
| Example 1 | 0.028 | 5.7 | p = 0.01 | 0.060 | 12.3 | p < 0.01 |
| * Comparative | | | | | | |

With the cream of Example 2 (placebo) there was not a statistically significant difference in skin elasticity (p>0.05). With the cream of Example 1 (of the invention) there was a statistically significant increase in skin elasticity ($p \leq 0.01$), both after 15 minutes and after 2 weeks.
In particular there was a remarkable 12.3% increase in skin elasticity after 2 weeks.

**Claims**

1. Skin care composition comprising from 0.05 to 5.0% by weight of a depolymerized hydroxypropyl guar (HPG) having molar substitution comprised between 1.5 and 3.5 and RVT Brookfield® viscosity at 10 % by weight in water, 20 °C, 20 rpm from 50 to 5000 mPa*s together with an acceptable cosmetic or pharmaceutical carrier or diluent.

2. The skin care composition according to claim 1, wherein said depolymerized HPG has a molar substitution comprised between 2.0 and 3.2.

3. The skin care composition according to claim 1 wherein said depolymerized HPG has RVT Brookfield® viscosity comprised between 100 and 2000 mPa*s at 20°C, 20 rpm and 10 % by weight in water.

4. The skin care composition according to claim 1 which is in the form of an aqueous or hydro-alcoholic system, an emulsion or a gel.

5. The skin care composition of claim 4, wherein said composition is an emulsion comprising from 0.1 to 15.0 % by weight of an emulsifier and from 1.0 to 60 % by weight of an oil.

6. The skin care composition of claim 5, wherein said oil is selected from the group consisting of hydrocarbon oils and waxes, silicone oils, fatty acid derivatives, cholesterol, cholesterol derivatives, diglycerides, triglycerides, vegetable oils, vegetable oil derivatives, alkyl or alkenyl esters, lanolin and its derivatives, wax esters, beeswax derivatives and sterols and combinations thereof.

7. The skin care composition of claim 5, wherein said emulsifier is selected from the group consisting of anionic, cationic, amphoteric, zwitterionic and nonionic emulsifiers; natural or synthetic polymeric emulsifiers; and mixtures thereof.

8. The skin care composition of claim 7, wherein said anionic emulsifiers are selected from the group consisting of alkyl and alkyl ether sulfates, alkyl sulfonates, alkyl and alkyl ether phosphates, alkyl or alkyl ether sulfosuccinates and alkyl and alkyl ether carboxylates or anionic derivatives of alkyl polyglycosides.

9. The skin care composition of claim 7, wherein said nonionic emulsifiers are selected from the group consisting of alkoxylated fatty alcohols or fatty acids, alkoxylated di- and tri-stiryl phenols, polyhydroxy fatty acid amides, sugar esters and polyesters, alkoxylated sugar esters, sorbitan and alkoxylated sorbitan fatty acid esters, alkyl polyglycosides and mixture thereof.

10. The skin care composition of claim 7, wherein said polymeric emulsifiers are crosslinked carboxylic acid polymers containing one or more monomers derived from acrylic acid, substituted acrylic acids, and salts and derivatives of these acrylic acids and substituted acrylic acids and/or ethylene oxide/propylene oxide block copolymers.

11. The skin care composition according to claim 1, which additionally comprises from 0.01 % to 30 % by weight of cosmetically-functional agents, selected from the group consisting of anti-wrinkle agents, antioxidant agent/radical scavengers, skin coolants, emollients, skin bleaching and lightening agents, sunscreen agents, tanning agents, skin soothing and healing agents, moisturizing agents, topical anesthetics, perfumes, deodorants other than perfumes, beauty aids, shaving actives, anti-acne agents, chelating agents, flavonoids, steroidal or non steroidal anti-inflammatory agents, antimicrobial agent, visual skin enhancer, and mixtures thereof.

12. The skin care composition according to claim 1, which additionally comprises conventional cosmetic additives selected from the group consisting of colorants, preservatives , antifoaming agents, nutritional supplements, activity enhancer, solubilizing agents, functional polymers, thickening agents, stabilizers, suspending agents, silicone material, hydrocarbon polymer, medicaments, and mixtures thereof.

13. The skin care composition according to any of the preceding claims, which is formulated as skin lotion, skin cream, body butter, after-shower lotion, after-cleansing lotion, cleansing milk, after-shave product, deodorant product, antiperspirant product, sun care preparation and self-tanning creams.

14. Use of a depolymerized hydroxypropyl guar having molar substitution comprised between 1.5 and 3.5 and RVT Brookfield® viscosity at 10 % by weight in water, 20 °C, 20 rpm from 50 to 5000 mPa*s for increasing the elasticity of the skin.

**Patentansprüche**

1. Hautpflegezusammensetzung, umfassend 0,05 bis 5,0 Gew.-% depolymerisiertes Hydroxypropyl-Guar (HPG) mit einer molaren Substitution zwischen 1,5 und 3,5 und einer Brookfield®-Viskosität RVT von 50 bis 5000 mPa.s bei 10 Gew.-% in Wasser, 20 °C, 20 UpM, zusammen mit einem verträglichen kosmetischen oder pharmazeutischen Träger oder Verdünnungsmittel.

2. Hautpflegezusammensetzung nach Anspruch 1, wobei das depolymerisierte HPG eine molare Substitution zwischen 2,0 und 3,2 hat.

3. Hautpflegezusammensetzung nach Anspruch 1, wobei das depolymerisierte HPG eine Brookfield®-Viskosität RVT zwischen 100 und 2000 mPa.s bei 20 °C, 20 UpM und 10 Gew.-% in Wasser hat.

4. Hautpflegezusammensetzung nach Anspruch 1, die in Form eines wässrigen oder wässrig-alkoholischen Systems, einer Emulsion oder eines Gels vorliegt.

5. Hautpflegezusammensetzung nach Anspruch 4, wobei die Zusammensetzung eine Emulsion ist, die 0,1 bis 15,0 Gew.-% Emulgator und 1,0 bis 60 Gew.-% Öl aufweist.

6. Hautpflegezusammensetzung nach Anspruch 5, wobei das Öl ausgewählt ist aus der Gruppe bestehend aus Kohlenwasserstoff-Ölen und -Wachsen, Silikonölen, Fettsäurederivaten, Cholesterol, Cholesterol-Derivaten, Diglycerlden, Triglyceriden, Pflanzenölen, Pflanzenöl-Derivaten, Alkyl- oder Alkenylestern, Lanolin und seinen Derivaten, Wachsestern, Bienenwachs-Derivaten und Sterolen und Kombinationen davon.

7. Hautpflegezusammensetzung nach Anspruch 5, wobei der Emulgator ausgewählt ist aus der Gruppe bestehend aus anionischen, kationischen, amphoteren, zwitterionischen und nicht-ionischen Emulgatoren; natürlichen oder synthetischen polymeren Emulgatoren; und Mischungen davon.

8. Hautpflegezusammensetzung nach Anspruch 7, wobei die anionischen Emulgatoren ausgewählt sind aus der Gruppe bestehend aus Alkyl- und Alkylethersulfaten, Alkylsulfonaten, Alkyl- und Alkyletherphosphaten, Alkyl- oder Alkylethersulfosuccinaten und Alkyl- und Alkylethercarhoxylaten oder anionischen Derivativen von Alkylpolyglycosiden.

9. Hautpflegezusammensetzung nach Anspruch 7, wobei die nicht-ionischen Emulgatoren ausgewählt sind aus der Gruppe bestehend aus alkoxylierten Fettalkoholen oder Fettsäuren, alkoxylierten Di- und Tristyrylphenolen, Polyhydroxyfettsäureamiden, Zuckerestern und -polyestern, alkoxylierten Zuckerestern, Sorbitan- und alkoxylierten Sorbitanfettsäureestern, Alkylpolyglycosiden und Mischungen davon.

10. Hautpflegezusammensetzung nach Anspruch 7, wobei die polymeren Emulgatoren vernetzte Carbonsäurepolymere, die ein oder mehrere Monomere aufweisen, die von Acrylsäure, substituierten Acrylsäuren und Salzen und Derivaten der Acrylsäuren und substituierten Acrylsäuren abgeleitet sind, und/oder Ethylenoxid/Propylenoxid-Blockcopolymere sind.

11. Hautpflegezusammensetzung nach Anspruch 1, die zusätzlich 0,01 bis 30 Gew.-% kosmetisch-funktioneller Mittel aufweist, ausgewählt aus der Gruppe bestehend aus Antifaltenmitteln, Antioxidanlien/Radikalfängern, haulkühlenden Mitteln, Emollientien, haulbleichenden und hautaufhellenden Mitteln, Sonnenschutzmitteln, Bräunungsmitteln, hautberuhigenden und heilenden Mitteln, feuchtigkeitsspendenden Mitteln, topischen Anästhetika, Parfums, von Parfums verschiedenen Deodorants, Schönheitsmitteln, Rasierhiffsmitteln, Mitteln gegen Akne, Chelatbildnern, Flavonoiden, steroidalen oder nichtsteroldalen Entzündungshemmern, antimikrobiellen Mitteln, Mitteln zur visuellen Hautverbesserung und Mischungen davon.

12. Hautpflegezusammensetzung nach Anspruch 1, die zusätzlich herkömmliche kosmetische Additive aufweist, ausgewählt aus der Gruppe bestehend aus Färbemitteln, Konservierungsmitteln, Antischaummitteln, Nährstoffsupplementen, Aktivitätsverstärkern, Lösungsvermittlern, funktionellen Polymeren, Verdickungsmitteln, Stabilisatoren, Suspendiermitteln, Silikonmaterial, Kohlenwasserstoffpolymeren, Medikamenten und Mischungen davon.

13. Hautpflegezusammensetzung nach einem der vorhergehenden Ansprüche, die als Hautlotion, Hautcreme, Körperbutter, Lotion zur Anwendung nach dem Duschen, Lotion zur Anwendung nach der Reinigung, Reinigungsmilch, Aftershave-Produkt, Deodorant-Produkt, Antitranspirant-Produkt, Sonnenschutz-Zubereitung und Selbstbräunungscreme formuliert ist.

14. Verwendung eines depolymerisierten Hydroxypropyl-Guar mit einer molaren Substitution zwischen 1,5 und 3,5 und einer Brookfield®-Viskosität RVT von 50 bis 5000 mPa.s bei 10 Gew.-% in Wasser, 20 °C, 20 UpM, zur Erhöhung der Hautelastizität.

## Revendications

1. Composition de soin pour la peau comprenant de 0,05 à 5,0 % en poids d'un hydroxypropylguar (HPG) dépolymérisé ayant une substitution molaire comprise entre 1,5 et 3,5 et une viscosité sur RVT Brookfield® à 10 % en poids dans de l'eau, 20 °C, 20 tr/min de 50 à 5 000 mPa.s avec un véhicule ou diluent cosmétiquement ou pharmaceutiquement acceptable.

2. Composition de soin pour la peau selon la revendication 1, dans laquelle ledit HPG dépolymérisé a une substitution molaire comprise entre 2,0 et 3,2.

3. Composition de soin pour la peau selon la revendication 1, dans laquelle ledit HPG dépolymérisé a une viscosité sur RVT Brookfield® comprise entre 100 et 2 000 mPa.s à 20 °C, 20 tr/min et 10 % en poids dans de l'eau.

4. Composition de soin pour la peau selon la revendication 1, qui est sous la forme d'un système aqueux ou hydro-alcoolique, d'une émulsion ou d'un gel.

5. Composition de soin pour la peau selon la revendication 4, dans laquelle ladite composition est une évulsion comprenant de 0,1 à 15,0 % en poids d'un émulsifiant et de 1,0 à 60 % en poids d'une huile.

6. Composition de soin pour la peau selon la revendication 5, dans laquelle ladite huile est choisie dans le groupe constitué par les huiles et les cires d'hydrocarbures, les huiles de silicone, les dérivés d'acides gras, le cholestérol, les dérivés du cholestérol, les diglycérides, les triglycérides, les huiles végétales, les dérivés d'huile végétale, les esters d'alkyle ou d'alcényle, la lanoline et ses dérivés, les esters de cire, les dérivés de cire d'abeille et les stérols et des combinaisons de ceux-ci.

7. Composition de soin pour la peau selon la revendication 5, dans laquelle ledit émulsifiant est choisi dans le groupe constitué par les émulsifiants anioniques, cationiques, amphotères, zwittérioniques et non ioniques ; les émulsifiants polymères naturels ou synthétique ; et des mélanges de ceux-ci.

8. Composition de soin pour la peau selon la revendication 7, dans laquelle lesdits émulsifiants anioniques sont choisis dans le groupe constitué par les sulfates d'alkyle et d'éther allylique, les sulfonates, d'alkyle, les phosphates d'alkyle et d'éther alkylique, les sulfosuccinates d'alkyle ou d'éther alkylique et les carboxylases d'alkyle et d'éther alkylique ou des dérivés anioniques d'alkylpolyglycosides.

9. Composition de soin pour la peau selon la revendication 7, dans laquelle lesdits émulsifiants non ioniques sont choisis dans le groupe constitué par les alcools gras ou les acides gras alcoxylés, les di- et tristirylphénols alcoxylés, les polyhydroxyamides d'acide gras, les esters et les polyesters de sucre, les esters de sucre alcoxylés, les esters d'acide gras de sorbitan et de sorbitan alcoxylé, les alkylpolyglycosides, et des mélanges de ceux-ci.

10. Composition de soin pour la peau selon la revendication 7, dans laquelle lesdits émulsifiants polymères sont des polymères d'acide carboxylique réticulés contenant un ou plusieurs monomères dérivés de l'acide acrylique, des acides acryliques substitués, et des sels et des dérivés de ces acides acryliques et acides acryliques substitués et/ou des copolymères séquence d'oxyde d'éthylène/oxyde de propylène.

11. Composition de soin pour la peau selon la revendication 1, qui comprend en outre de 0,01 % à 30 % en poids d'agents cosmétiquement fonctionnels choisis dans le groupe constitué par les agents anti-rides, les agents antioxydants/piégeurs de radicaux, les rafraîchissants cutanés, les émollients, les agents de blanchiment et d'éclaircissement de la peau, les agents de protection solaire, les agents bronzants, les agents apaisants et cicatrisants, les agents hydratants, les anesthésiques topiques, les parfums, les déodorants autres que les parfums, les adjuvants de beauté, les agents actifs de rasage, les agents anti-acné, les agents chélatants, les flavonoïdes, les agents anti-inflammatoires stéroidiens ou non stéroidiens, les agents antimicrobiens, les améliorateurs cutanés visuels, et des mélanges de ceux-ci.

12. Composition de soin pour la peau selon la revendication 1, qui comprend en outre des additifs cosmétiques classiques choisis dans le groupe constitué par les colorants, les conservateurs, les agents antimousse, les compléments nutritionnels, les améliorateurs d'activité, les agents solubilisants, les polymères fonctionnels, les agents épaississants, les stabilisateurs, les agents de suspension, une matière silicone, un polymère hydrocarboné, des médicaments, et des mélanges de ceux-ci.

13. Composition de soin pour la peau selon l'une quelconque des revendications précédentes, qui est formulée sous forme de lotion cutanée, crème cutanée, beurre corporel, lotion après-douche, lotion après-nettoyage, lait nettoyant, produit après-rasage, produit déodorant, produit anti-transpirant, préparation de protection solaire et crèmes autobronzantes.

14. Utilisation d'un hydroxypropylguar dépolymérisé ayant une substitution molaire comprise entre 1,5 et 3,5 et une viscosité sur RVT Brookfield® à 10 % en poids dans de l'eau, 20 °C, 20 tr/min de 50 à 5 000 mPa.s pour une augmentation de l'élasticité de la peau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3728331 A
- US 4874854 A
- US 5708162 A
- US 4753659 A
- US 6884884 B
- EP 030443 A
- EP 1417240 A
- JP 7100017 B
- WO 9315116 A
- WO 9904027 A

**Non-patent literature cited in the description**

- **CRAIG, D. et al.** Proceedings of the Thirty-Ninth Annual Southwestern Petroleum Short Course. Southwestern Petroleum Short Course Association, Inc, 22 April 1992
- **VIJAYENDRAN ; BONE.** *Carbohydrate Polymers,* 1994, vol. 4, 299-313
- **FROLLINI, E. et al.** *Carbohydrate Polymers,* 1995, vol. 27, 129-135
- **OUCHI, T. et al.** *J.M.S.-Pure Appl. Chem.,* 1997, vol. A34 (6), 975-989
- **TAYAL, A. et al.** *Macromolecules,* 2000, vol. 33, 9488-9484
- **WHISTLER, ROY, L ; BEMILLER, JAMES N.** Industrial Gums: Polysaccharides and their Derivatives. Academic Press, 1993
- McCutcheon's Detergents and Emulsifiers. 2003